# EUROPEAN PATENT APPLICATION

(11) **EP 4 752 548 A1**
(43) Date of publication of application: **03.06.2026**
(21) Application number: 24844932.4
(22) Date of filing: 24.07.2024
(51) Int. Cl.: G01N 33/573

(54) **BIOMARKERS FOR RESPONSE TO BARIATRIC SURGERY**

(30) Priority: 26.07.2023 ES 202330641
(71) Applicant: Universidad de Córdoba, 14005 Córdoba (ES); Servicio Andaluz de Salud, 41071 Sevilla (ES)
(72) Inventor: GUZMÁN RUIZ, Rocío, 14004 Córdoba Córdoba (ES); MALAGÓN POYATO, María Del Mar, 14004 Córdoba Córdoba (ES); SÁNCHEZ CEINOS, Julia, 14004 Córdoba Córdoba (ES); TERCERO ALCÁZAR, Carmen, 14004 Córdoba Córdoba (ES); CANO GONZÁLEZ, David, 41013 Sevilla Sevilla (ES); GARCÍA LUNA, Pedro Pablo, 41013 Sevilla Sevilla (ES); PEREIRA CUNILL, José Luis, 41013 Sevilla Sevilla (ES); MORALES CONDE, Salvador, 41013 Sevilla Sevilla (ES)
(74) Representative: San Martin Alarcia, Esther
(86) International application number: PCT/ES2024/070478
(87) International publication number: WO 2025/022039

(57) **Abstract**

The invention relates to the *in vitro* use of various molecular biomarker levels measured in samples from subjects with obesity, said samples being isolated before the bariatric surgery, as biomarkers for predicting or prognosing the response to bariatric surgery.

## Description

### TECHNICAL FIELD

The present invention relates to the field of medicine, specifically to the field of medical prognosis and diagnosis, and even more specifically relates to the use of biomarkers for predicting the response of subjects undergoing bariatric surgery.

### STATE OF THE ART

Obesity is a chronic disease that is characterized by the increase in body adipose tissue and by a low-grade chronic inflammation state that predisposes to the development of a series of associated comorbidities, such as dyslipidemia, arterial hypertension and/or type 2 diabetes, which are encompassed in the so-called metabolic syndrome.

Bariatric surgery is currently the most effective treatment for the long-term control of morbid obesity and metabolic syndrome, above dietary treatment, lifestyle modifications, exercise, behavioral therapy, and drug treatment, as this type of therapies exhibit high recurrence rates, achieving insufficient weight losses for a significant improvement of comorbidities. While this is an invasive procedure, bariatric surgery has proven to be a safe and effective technique, currently showing a mortality rate of less than 0.5%. The main benefit of bariatric surgery is a significant decrease in body weight, which is accompanied by a decrease in adipose tissue and an improvement of the associated comorbidities, primarily of carbohydrate metabolism. However, it can be associated with a number of potentially serious complications, both early (anastomotic leaks, gastrointestinal bleeding, deep vein thrombosis, pulmonary thromboembolism, intestinal obstruction, surgical incision infection, pneumonia, and cardiac events) as late (marginal ulcers, internal hernias, dumping syndrome, cholecystitis, anemia, and osteomalacia).

A great variability in the response of subjects to surgery has been observed, not obtaining the same benefits in improving obesity and its co-morbidities in all of them. Some subjects experience insufficient weight loss and reduction of insufficient cardiovascular risk factors, as well as the persistence of type 2 diabetes.

The exact causes of this resistance to bariatric surgery are unknown, although many preoperative factors have been proposed as predictors (negative or positive) based on body weight reduction or remission of diabetes after surgery. Some factors that may favor their occurrence, such as age and plasma levels of inflammatory cytokines, have also been identified. However, none of these factors have consistently proven to have sufficiently strong predictive power to influence eligibility for bariatric surgery itself and predictive molecular markers of bariatric surgery have not been identified that allow more specific strategies and inclusion criteria to be designed.

Therefore, it would be very useful to have markers of predictive response markers to weight loss induced by bariatric surgery in order to ensure a suitable situation for each subject. To date, the initial state of the adipose tissue has not been taken into account, which could be a key point in the variability of the observed response. Moreover, the main organ affected in bariatric surgery, since the majority of the percentage of loss associated with the procedure is due to a decrease in said tissue, knowing the previous state of the adipose tissue before the procedure could be considered a criterion for evaluating and selecting subjects as candidates for bariatric surgery. Therefore, having factors that predict the response to bariatric surgery based on the initial state of the adipose tissue could be a key tool in clinical practice that allows improving the selection of subjects by identifying those in which more exhaustive or alternative therapies must be applied.

The most comprehensive approach to this goal comes from the field of proteomics. Because proteins reflect the underlying cellular biological processes, studying the proteome of adipose tissue could allow the exploration of the biological and pathophysiological mechanisms involved in the relationship between obesity and its associated comorbidities. The use of "molecular fingerprints" obtained from proteomic studies are widely used and very useful tools in the diagnosis of numerous pathologies. Thus, the identification of a molecular fingerprint capable of predicting the state of the adipose tissue could be a possible preoperative prediction tool, which, together with the other criteria already established for bariatric surgery, allows reducing the variability in the improvement response, increasing the success rates of said intervention.

### DESCRIPTION OF THE INVENTION

The object of the present invention is to establish preoperative parameters that allow the prediction of responsiveness to bariatric surgery for weight loss. In this sense, a molecular biomarker of preference has been defined, CYB5 (cytochrome b5, form A) and a panel of molecular biomarkers including it, whose preoperative levels of expression in subcutaneous adipose tissue, allow to predict the response to bariatric surgery. The panel of molecular biomarkers consists of the following biomarkers:

**Table 1: Biomarkers of the invention panel.**

| | | | | |
|---|---|---|---|---|
| CYB5 | SUCB2 | HBA | QCR1 | GRP78 |
| SYUG | LRP1 | GPX4 | SURF4 | AIFM1 |

CYB5, Cytochrome b5 type A; SYUG, Synuclein Gamma; HBA, Hemoglobin Subunit Alpha; GPX4, Glutathione Peroxidase 4; GRP78, 78 kDa glucose-regulated protein; SUCB2, Succinate-CoA Ligase GDP-forming Subunit Beta, mitochondrial; LRP1, Low Density Lipoprotein Receptor-Related Protein 1; QCR1, Cytochrome b-c1 complex subunit 1, mitochondrial; SURF4, excess locus 4 protein; and AIFM1, Apoptosis-inducing factor 1, mitochondrial.

Likewise, the level of expression of oxidized LDL has been determined as a surrogate plasma marker of CYB5 that also allows predicting said response to bariatric surgery.

Therefore, a first aspect of the invention relates to the *in vitro* use of the level of expression of CYB5 in a sample of subcutaneous adipose tissue from a subject with obesity candidate to undergo bariatric surgery, isolated prior to the bariatric surgical procedure, as a biomarker for predicting or prognosing the response to bariatric surgery, where under-expressed levels with respect to the average reference values of a group of subjects with obesity who are candidates to undergo bariatric surgery is an indicator of belonging to the group of subjects presenting moderate response to bariatric surgery.

Note that, although there are more or less universally accepted criteria in the medical field for establishing obesity levels based on an individual's BMI values (such as that an individual with a BMI of 25 or above is overweight and an individual with a BMI of 30 presents obesity) the criteria for deciding whether a subject is eligible for bariatric surgery are not as clear, or universal.

In the context of the present invention, "a subject with obesity candidate to undergo bariatric surgery" is defined as an individual who can benefit from the effects derived from bariatric surgery, and is therefore a candidate to be subjected to said surgery.

The beneficial effects of bariatric surgery range from weight loss and the resulting reduction in BMI, until the improvement of certain obesity-associated clinical parameters such as those that measure blood glucose control (blood glucose or insulin levels) or lipid metabolism (cholesterol, HDL, and LDL levels). Other co-morbidities associated with obesity such as hypertension, dilated cardiomyopathy, or sleep apnea may also be enhanced.

Thus, medical personnel may consider individuals with a BMI above 30 and comorbidities that may be enhanced by surgery as candidates for bariatric surgery, so these individuals would enter part of "the subjects with obesity who are candidates to undergo bariatric surgery". Preferably, a "subject with obesity who is candidate to undergo bariatric surgery" is one having a BMI above 35 and comorbidities, or having a BMI above 40 without mediating comorbidities. In the present invention, within the "conditions associated with obesity", diabetes, hypertension, dilated cardiomyopathy or sleep apnea are highlighted.

In the present invention "good response" is defined as a loss of Body Mass Index (BMI) of the subject, 15 months after the intervention, equal to or greater than 30% in relation to the BMI prior to the intervention. In the present invention "moderate response" is defined as a loss of Body Mass Index (BMI) of the subject, 15 months after the intervention, less than 30% in relation to the BMI prior to the intervention. This value of 30% BMI loss is established in relation to the median BMI loss of a group of subjects who underwent bariatric surgery 15 months after the intervention. Therefore, in the present invention "good response" is defined as a loss of Body Mass Index (BMI) of the subject 15 months after intervention, equal to or greater than the median BMI loss of the group of subjects who underwent bariatric surgery after the same period of time. Thus, "moderate response" is defined as a loss of Body Mass Index (BMI) of the subject 15 months after the procedure that is less than the median BMI loss of the group of subjects who underwent bariatric surgery after the same period of time.

In a preferred embodiment of this aspect of the invention, this biomarker, the level of expression of CYB5, is used in combination with the level of expression of SYUG, HBA, GPX4, GRP78, SUCB2, LRP1, QCR1, SURF4 and AIFM1, all measured in a sample of the subcutaneous adipose tissue from subjects with obesity isolated prior to the bariatric surgery, generating a panel of biomarkers for predicting or prognosing the response to bariatric surgery in subjects with obesity, wherein under-expressed levels of CYB5, SUCB2, QCR1, SURF4, GRP78, GPX4, AIFM1 and HBA, and overexpressed levels of LRP1 and SYUG with respect to the average values of an individual with obesity that exhibits good response to bariatric surgery are indicators of belonging to the group of subjects presenting moderate response to bariatric surgery.

Another aspect of the invention relates to the *in vitro* use of the level of expression of oxidized LDL in an isolated blood, serum or plasma sample from subjects with obesity, this being a surrogate marker of CYB5, as a biomarker for predicting or prognosing the response to bariatric surgery in subjects with obesity, wherein overexpressed levels with respect to the average values of an individual with obesity that has good response to bariatric surgery is an indicator of belonging to the group of subjects presenting moderate response to bariatric surgery.

In the context of the present invention, CYB5 is defined as the amino acid molecules corresponding to NCBI reference sequences:
NP_683725.1 - Isoform 1, with sequence SEQ ID NO:1: 
MAEQSDEAVKYYTLEEIQKHNHSKSTWLILHHKVYDLTKFLEEHPGGEEVLREQAGGDA
TENFEDVGHSTDAREMSKTFIIGELHPDDRPKLNKPPETLITTIDSSSSWWTNWVIPAISA
VAVALMYRLYMAED,
NP_001905.1 - Isoform 2, with sequence SEQ ID NO:2: 

, or
NP_001177736.1- Isoform 3, with sequence SEQ ID NO:3:

MAEQSDEAVKYYTLEEIQKHNHSKSTWLILHHKVYDLTKFLEEHPGGEEVLREQAGGDA
TENFEDVGHSTDAREMSKTFIIGELHPETLITTIDSSSSWWTNWVIPAISAVAVALMYRLY
MAED.

In the context of the present invention, SYUG is defined as the amino acid molecules corresponding to NCBI reference sequence: NP_003078.2 of sequence SEQ ID NO:4:

MDVFKKGFSIAKEGVVGAVEKTKQGVTEAAEKTKEGVMYVGAKTKENVVQSVTSVAEKT
KEQANAVSEAVVSSVNTVATKTVEEAENIAVTSGVVRKEDLRPSAPQQEGEASKEKEEV
AEEAQSGGD.

In the context of the present invention, HBA is defined as the amino acid molecules corresponding to NCBI reference sequence: NP_000508.1 of sequence SEQ ID NO:5:



.

In the context of the present invention, GPX4 is defined as the amino acid molecules corresponding to the reference sequences:
NCBI: NP_002076.2 - Isoform A, sequence SEQ ID NO:6: 
MSLGRLCRLLKPALLCGALAAPGLAGTMCASRDDWRCARSMHEFSAKDIDGHMVNLDK
YRGFVCIVTNVASQUGKTEVNYTQLVDLHARYAECGLRILAFPCNQFGKQEPGSNEEIKE
FAAGYNVKFDMFSKICVNGDDAHPLWKWMKIQPKGKGILGNAIKWNFTKFLIDKNGCVV
KRYGPMEEPLVIEKDLPHYF,
NCBI: NP_001034936.1- Isoform B, sequence SEQ ID NO:7: 
MSLGRLCRLLKPALLCGALAAPGLAGTMCASRDDWRCARSMHEFSAKDIDGHMVNLDK
YRGFVCIVTNVASQUGKTEVNYTQLVDLHARYAECGLRILAFPCNQFGKQEPGSNEEIKE
FAAGYNVKFDMFSKICVNGDDAHPLWKWMKIQPKGKGILGNAIKWNFTKFGHRLSTVPH
RQERLRGEALRTHGGAPGDREGPAPLFLAPQVCGPARAPAHALGAFHRHS,
NCBI: NP_001034937.1- Isoform C, sequence SEQ ID NO:8: 
MGRAGAGSPGRRRQRCQSRGRRRPRAPRRRKAPACRRRARRRRKKPCPRSLRPEI
HECPKSQDPCASRDDWRCARSMHEFSAKDIDGHMVNLDKYRGFVCIVTNVASQUGKTE
VNYTQLVDLHARYAECGLRILAFPCNQFGKQEPGSNEEIKEFAAGYNVKFDMFSKICVNG
DDAHPLWKWMKIQPKGKGILGNAIKWNFTKFLIDKNGCVVKRYGPMEEPLVIEKDLPHYF
 , or
NCBI: NP_001354761.1- Isoform D, sequence SEQ ID NO:9:

MCASRDDWRCARSMHEFSAKDIDGHMVNLDKYRGFVCIVTNVASQUGKTEVNYTQLVD
LHARYAECGLRILAFPCNQFGKQEPGSNEEIKEFAAGYNVKFDMFSKICVNGDDAHPLW
KWMKIQPKGKGILGNAIKWNFTKFLIDKNGCVVKRYGPMEEPLVIEKDLPHYF.

In the context of the present invention, GRP78 is defined as the amino acid molecules corresponding to NCBI reference sequence: NP_005338.1 of sequence SEQ ID NO:10:

MKLSLVAAMLLLLSAARAEEEDKKEDVGTVVGIDLGTTYSCVGVFKNGRVEIIANDQGNRI
TPSYVAFTPEGERLIGDAAKNQLTSNPENTVFDAKRLIGRTWNDPSVQQDIKFLPFKVVE
KKTKPYIQVDIGGGQTKTFAPEEISAMVLTKMKETAEAYLGKKVTHAVVTVPAYFNDAQR
QATKDAGTIAGLNVMRIINEPTAAAIAYGLDKREGEKNILVFDLGGGTFDVSLLTIDNGVFE
VVATNGDTHLGGEDFDQRVMEHFIKLYKKKTGKDVRKDNRAVQKLRREVEKAKRALSS
QHQARIEIESFYEGEDFSETLTRAKFEELNMDLFRSTMKPVQKVLEDSDLKKSDIDEIVLV
GGSTRIPKIQQLVKEFFNGKEPSRGINPDEAVAYGAAVQAGVLSGDQDTGDLVLLDVCPL
TLGIETVGGVMTKLIPRNTVVPTKKSQIFSTASDNQPTVTIKVYEGERPLTKDNHLLGTFDL
TGIPPAPRGVPQIEVTFEIDVNGILRVTAEDKGTGNKNKITITNDQNRLTPEEIERMVNDAE
KFAEEDKKLKERIDTRNELESYAYSLKNQIGDKEKLGGKLSSEDKETMEKAVEEKIEWLE
SHQDADIEDFKAKKKELEEIVQPIISKLYGSAGPPPTGEEDTAEKDEL.

In the context of the present invention, SUCB2 is defined as the amino acid molecules corresponding to the reference sequences:
NCBI: NP_001171070.1 of sequence SEQ ID NO:11: 
MASPVAAQAGKLLRALALRPRFLAAGSQAVQLTSRRWLNLQEYQSKKLMSDNGVRVQR
FFVADTANEALEAAKRLNAKEIVLKAQILAGGRGKGVFNSGLKGGVHLTKDPNVVGQLAK
QMIGYNLATKQTPKEGVKVNKVMVAEALDISRETYLAILMDRSCNGPVLVGSPQGGVDIE
 
EVAASNPELIFKEQIDIFEGIKDSQAQRMAENLGFVGPLKSQAADQITKLYNLFLKIDATQV
EVNPFGETPEGQVVCFDAKINFDDNAEFRQKDIFAMDDKSENEPIENEAAKYDLKYIGLD
GNIACFVNGAGLAMATCDIIFLNGGKPANFLDLGGGVKEAQVYQAFKLLTADPKVEAILVN
IFGGIVNCAIIANGITKACRELELKVPLVVRLEGTFMEKKGSYMHIKQETGNSNENITGIQE
NVAHQNLSKCAISIFLC, or
NCBI: NP_003839.2 Isoform 2, sequence SEQ ID NO:12:

MASPVAAQAGKLLRALALRPRFLAAGSQAVQLTSRRWLNLQEYQSKKLMSDNGVRVQR
FFVADTANEALEAAKRLNAKEIVLKAQILAGGRGKGVFNSGLKGGVHLTKDPNVVGQLAK
QMIGYNLATKQTPKEGVKVNKVMVAEALDISRETYLAILMDRSCNGPVLVGSPQGGVDIE
EVAASNPELIFKEQIDIFEGIKDSQAQRMAENLGFVGPLKSQAADQITKLYNLFLKIDATQV
EVNPFGETPEGQVVCFDAKINFDDNAEFRQKDIFAMDDKSENEPIENEAAKYDLKYIGLD
GNIACFVNGAGLAMATCDIIFLNGGKPANFLDLGGGVKEAQVYQAFKLLTADPKVEAILVN
IFGGIVNCAIIANGITKACRELELKVPLVVRLEGTNVQEAQKILNNSGLPITSAIDLEDAAKK
AVASVAK.

In the context of the present invention, LRP1 is defined as the amino acid molecules corresponding to NCBI reference sequence: NP_002323.2 of sequence SEQ ID

NO:13:MLTPPLLLLLPLLSALVAAAIDAPKTCSPKQFACRDQITCISKGWRCDGERDCPDG
SDEAPEICPQSKAQRCQPNEHNCLGTELCVPMSRLCNGVQDCMDGSDEGPHCRELQG
NCSRLGCQHHCVPTLDGPTCYCNSSFQLQADGKTCKDFDECSVYGTCSQLCTNTDGSF
ICGCVEGYLLQPDNRSCKAKNEPVDRPPVLLIANSQNILATYLSGAQVSTITPTSTRQTTA
MDFSYANETVCWVHVGDSAAQTQLKCARMPGLKGFVDEHTINISLSLHHVEQMAIDWLT
GNFYFVDDIDDRIFVCNRNGDTCVTLLDLELYNPKGIALDPAMGKVFFTDYGQIPKVERC
DMDGQNRTKLVDSKIVFPHGITLDLVSRLVYWADAYLDYIEVVDYEGKGRQTIIQGILIEHL
YGLTVFENYLYATNSDNANAQQKTSVIRVNRFNSTEYQVVTRVDKGGALHIYHQRRQPR
VRSHACENDQYGKPGGCSDICLLANSHKARTCRCRSGFSLGSDGKSCKKPEHELFLVY
GKGRPGIIRGMDMGAKVPDEHMIPIENLMNPRALDFHAETGFIYFADTTSYLIGRQKIDGT
ERETILKDGIHNVEGVAVDWMGDNLYWTDDGPKKTISVARLEKAAQTRKTLIEGKMTHP
RAIVVDPLNGWMYWTDWEEDPKDSRRGRLERAWMDGSHRDIFVTSKTVLWPNGLSLDI
PAGRLYWVDAFYDRIETILLNGTDRKIVYEGPELNHAFGLCHHGNYLFWTEYRSGSVYRL
ERGVGGAPPTVTLLRSERPPIFEIRMYDAQQQQVGTNKCRVNNGGCSSLCLATPGSRQ
CACAEDQVLDADGVTCLANPSYVPPPQCQPGEFACANSRCIQERWKCDGDNDCLDNS
DEAPALCHQHTCPSDRFKCENNRCIPNRWLCDGDNDCGNSEDESNATCSARTCPPNQ
FSCASGRCIPISWTCDLDDDCGDRSDESASCAYPTCFPLTQFTCNNGRCININWRCDND
NDCGDNSDEAGCSHSCSSTQFKCNSGRCIPEHWTCDGDNDCGDYSDETHANCTNQAT
RPPGGCHTDEFQCRLDGLCIPLRWRCDGDTDCMDSSDEKSCEGVTHVCDPSVKFGCK

DSARCISKAWVCDGDNDCEDNSDEENCESLACRPPSHPCANNTSVCLPPDKLCDGNDD
CGDGSDEGELCDQCSLNNGGCSHNCSVAPGEGIVCSCPLGMELGPDNHTCQIQSYCA
KHLKCSQKCDQNKFSVKCSCYEGWVLEPDGESCRSLDPFKPFIIFSNRHEIRRIDLHKGD
YSVLVPGLRNTIALDFHLSQSALYWTDVVEDKIYRGKLLDNGALTSFEVVIQYGLATPEGL
AVDWIAGNIYWVESNLDQIEVAKLDGTLRTTLLAGDIEHPRAIALDPRDGILFWTDWDASL
PRIEAASMSGAGRRTVHRETGSGGWPNGLTVDYLEKRILWIDARSDAIYSARYDGSGHM
EVLRGHEFLSHPFAVTLYGGEVYWTDWRTNTLAKANKWTGHNVTVVQRTNTQPFDLQV
YHPSRQPMAPNPCEANGGQGPCSHLCLINYNRTVSCACPHLMKLHKDNTTCYEFKKFLL
YARQMEIRGVDLDAPYYNYIISFTVPDIDNVTVLDYDAREQRVYWSDVRTQAIKRAFINGT
GVETVVSADLPNAHGLAVDWVSRNLFWTSYDTNKKQINVARLDGSFKNAVVQGLEQPH
GLVVHPLRGKLYWTDGDNISMANMDGSNRTLLFSGQKGPVGLAIDFPESKLYWISSGNH
TINRCNLDGSGLEVIDAMRSQLGKATALAIMGDKLWWADQVSEKMGTCSKADGSGSVV
LRNSTTLVMHMKVYDESIQLDHKGTNPCSVNNGDCSQLCLPTSETTRSCMCTAGYSLRS
GQQACEGVGSFLLYSVHEGIRGIPLDPNDKSDALVPVSGTSLAVGIDFHAENDTIYWVDM
GLSTISRAKRDQTWREDVVTNGIGRVEGIAVDWIAGNIYWTDQGFDVIEVARLNGSFRYV
VISQGLDKPRAITVHPEKGYLFWTEWGQYPRIERSRLDGTERVVLVNVSISWPNGISVDY
QDGKLYWCDARTDKIERIDLETGENREVVLSSNNMDMFSVSVFEDFIYWSDRTHANGSI
KRGSKDNATDSVPLRTGIGVQLKDIKVFNRDRQKGTNVCAVANGGCQQLCLYRGRGQR
ACACAHGMLAEDGASCREYAGYLLYSERTILKSIHLSDERNLNAPVQPFEDPEHMKNVIA
LAFDYRAGTSPGTPNRIFFSDIHFGNIQQINDDGSRRITIVENVGSVEGLAYHRGWDTLY
WTSYTTSTITRHTVDQTRPGAFERETVITMSGDDHPRAFVLDECQNLMFWTNWNEQHP
SIMRAALSGANVLTLIEKDIRTPNGLAIDHRAEKLYFSDATLDKIERCEYDGSHRYVILKSE
PVHPFGLAVYGEHIFWTDWVRRAVQRANKHVGSNMKLLRVDIPQQPMGIIAVANDTNSC
ELSPCRINNGGCQDLCLLTHQGHVNCSCRGGRILQDDLTCRAVNSSCRAQDEFECANG
ECINFSLTCDGVPHCKDKSDEKPSYCNSRRCKKTFRQCSNGRCVSNMLWCNGADDCG
DGSDEIPCNKTACGVGEFRCRDGTCIGNSSRCNQFVDCEDASDEMNCSATDCSSYFRL
GVKGVLFQPCERTSLCYAPSWVCDGANDCGDYSDERDCPGVKRPRCPLNYFACPSGR
CIPMSWTCDKEDDCEHGEDETHCNKFCSEAQFECQNHRCISKQWLCDGSDDCGDGSD
EAAHCEGKTCGPSSFSCPGTHVCVPERWLCDGDKDCADGADESIAAGCLYNSTCDDRE
FMCQNRQCIPKHFVCDHDRDCADGSDESPECEYPTCGPSEFRCANGRCLSSRQWECD
GENDCHDQSDEAPKNPHCTSQEHKCNASSQFLCSSGRCVAEALLCNGQDDCGDSSDE
RGCHINECLSRKLSGCSQDCEDLKIGFKCRCRPGFRLKDDGRTCADVDECSTTFPCSQR
CINTHGSYKCLCVEGYAPRGGDPHSCKAVTDEEPFLIFANRYYLRKLNLDGSNYTLLKQG
LNNAVALDFDYREQMIYWTDVTTQGSMIRRMHLNGSNVQVLHRTGLSNPDGLAVDWVG
GNLYWCDKGRDTIEVSKLNGAYRTVLVSSGLREPRALVVDVQNGYLYWTDWGDHSLIG
RIGMDGSSRSVIVDTKITWPNGLTLDYVTERIYWADAREDYIEFASLDGSNRHVVLSQDIP
HIFALTLFEDYVYWTDWETKSINRAHKTTGTNKTLLISTLHRPMDLHVFHALRQPDVPNH

PCKVNNGGCSNLCLLSPGGGHKCACPTNFYLGSDGRTCVSNCTASQFVCKNDKCIPFW
WKCDTEDDCGDHSDEPPDCPEFKCRPGQFQCSTGICTNPAFICDGDNDCQDNSDEAN
CDIHVCLPSQFKCTNTNRCIPGIFRCNGQDNCGDGEDERDCPEVTCAPNQFQCSITKRCI
PRVWVCDRDNDCVDGSDEPANCTQMTCGVDEFRCKDSGRCIPARWKCDGEDDCGDG
SDEPKEECDERTCEPYQFRCKNNRCVPGRWQCDYDNDCGDNSDEESCTPRPCSESEF
SCANGRCIAGRWKCDGDHDCADGSDEKDCTPRCDMDQFQCKSGHCIPLRWRCDADA
DCMDGSDEEACGTGVRTCPLDEFQCNNTLCKPLAWKCDGEDDCGDNSDENPEECARF
VCPPNRPFRCKNDRVCLWIGRQCDGTDNCGDGTDEEDCEPPTAHTTHCKDKKEFLCR
NQRCLSSSLRCNMFDDCGDGSDEEDCSIDPKLTSCATNASICGDEARCVRTEKAAYCAC
RSGFHTVPGQPGCQDINECLRFGTCSQLCNNTKGGHLCSCARNFMKTHNTCKAEGSEY
QVLYIADDNEIRSLFPGHPHSAYEQAFQGDESVRIDAMDVHVKAGRVYWTNWHTGTISY
RSLPPAAPPTTSNRHRRQIDRGVTHLNISGLKMPRGIAIDWVAGNVYWTDSGRDVIEVAQ
MKGENRKTLISGMIDEPHAIVVDPLRGTMYWSDWGNHPKIETAAMDGTLRETLVQDNIQ
WPTGLAVDYHNERLYWADAKLSVIGSIRLNGTDPIVAADSKRGLSHPFSIDVFEDYIYGVT
YINNRVFKIHKFGHSPLVNLTGGLSHASDVVLYHQHKQPEVTNPCDRKKCEWLCLLSPS
GPVCTCPNGKRLDNGTCVPVPSPTPPPDAPRPGTCNLQCFNGGSCFLNARRQPKCRC
QPRYTGDKCELDQCWEHCRNGGTCAASPSGMPTCRCPTGFTGPKCTQQVCAGYCAN
NSTCTVNQGNQPQCRCLPGFLGDRCQYRQCSGYCENFGTCQMAADGSRQCRCTAYF
EGSRCEVNKCSRCLEGACVVNKQSGDVTCNCTDGRVAPSCLTCVGHCSNGGSCTMNS
KMMPECQCPPHMTGPRCEEHVFSQQQPGHIASILIPLLLLLLLVLVAGVVFWYKRRVQG
AKGFQHQRMTNGAMNVEIGNPTYKMYEGGEPDDVGGLLDADFALDPDKPTNFTNPVYA
TLYMGGHGSRHSLASTDEKRELLGRGPEDEIGDPLA.

In the context of the present invention, QCR1 is defined as the amino acid molecules corresponding to NCBI reference sequence: NP_003356.2 of sequence SEQ ID NO:14:

MAASVVCRAATAGAQVLLRARRSPALLRTPALRSTATFAQALQFVPETQVSLLDNGLRV
ASEQSSQPTCTVGVWIDVGSRFETEKNNGAGYFLEHLAFKGTKNRPGSALEKEVESMG
AHLNAYSTREHTAYYIKALSKDLPKAVELLGDIVQNCSLEDSQIEKERDVILREMQENDAS
MRDVVFNYLHATAFQGTPLAQAVEGPSENVRKLSRADLTEYLSTHYKAPRMVLAAAGGV
EHQQLLDLAQKHLGGIPWTYAEDAVPTLTPCRFTGSEIRHRDDALPFAHVAIAVEGPGW
ASPDNVALQVANAIIGHYDCTYGGGVHLSSPLASGAVANKLCQSFQTFSICYAETGLLGA
HFVCDRMKIDDMMFVLQGQWMRLCTSATESEVARGKNILRNALVSHLDGTTPVCEDIGR
SLLTYGRRIPLAEWESRIAEVDASVVREICSKYIYDQCPAVAGYGPIEQLPDYNRIRSGMF
WLRF.

In the context of the present invention, SURF4 is defined as the amino acid molecules corresponding to the reference sequences
NCBI: NP_001267717.1 - Isoform 2, sequence SEQ ID NO:15: 
MFLRVTKQYLPHVARLCLISTFLEDGIRMWFQWSEQRDYIDTTWNCGYLLASSFVFLNLL
GQLTGCVLVLSRNFVQYACFGLFGIIALQTIAYSILWDLKFLMRNLALGGGLLLLLAESRSE
GKSMFAGVPTMRESSPKQYMQLGGRVLLVLMFMTLLHFDASFFSIVQNIVGTALMILVAI
GFKTKLAALTLVVWLFAINVYFNAFWTIPVYKPMHDFLKYDFFQTMSVIGGLLLVVALGPG
GVSMDEKKKEW,
NCBI: NP_001267718.1 - Isoform 3, sequence SEQ ID NO:16: 
MGQNDLMGTAEDFADQFLRVTKQYLPHVARLCLISTFLEDGIRMWFQWSEQRDYIDTT
WNCGYLLASSFVFLNLLGQLTGCVLVLSRNFVQYACFGLFGIIALQTIAYSILWDLKFLMR
NLALGGGLLLLLAESRSEGKSMFAGVPTMRESSPKQYLPG,
NCBI: NP_001267719.1 - Isoform 4, sequence SEQ ID NO:17: 
MWFQWSEQRDYIDTTWNCGYLLASSFVFLNLLGQLTGCVLVLSRNFVQYACFGLFGIIAL
QTIAYSILWDLKFLMRNLALGGGLLLLLAESRSEGKSMFAGVPTMRESSPKQYMQLGGR
VLLVLMFMTLLHFDASFFSIVQNIVGTALMILVAIGFKTKLAALTLVVWLFAINVYFNAFWTI
PVYKPMHDFLKYDFFQTMSVIGGLLLVVALGPGGVSMDEKKKEW, or
NCBI: NP_001267721.1 - Isoform 5, sequence SEQ ID NO:18:

MGQNDLMGTAEDFADQFLRVTKQYLPHVARLCLISTFLEDGIRMWFQWSEQRDYIDTT
WNCGYLLASSFVFLNLLGQLTGCVLVLSRNFVQYACFGLFGIIALQTIAYSILWDLKFLMRL
SRTSWAQL.

In the context of the present invention, AIFM1 is defined as the amino acid molecules corresponding to NCBI reference sequence: NP_001124318.2 of sequence SEQ ID

NO:19:MGKILPEYLSNWTMEKVRREGVKVMPNAIVQSVGVSSGKLLIKLKDGRKVETDHI
VAAVGLEPNVELAKTGGLEIDSDFGGFRVNAELQARSNIWVAGDAACFYDIKLGRRRVE
HHDHAVVSGRLAGENMTGAAKPYWHQSMFWSDLGPDVGYEAIGLVDSSLPTVGVFAK
ATAQDNPKSATEQSGTGIRSESETESEASEITIPPSTPAVPQAPVQGEDYGKGVIFYLRD
KVVVGIVLWNIFNRMPIARKIIKDGEQHEDLNEVAKLFNIHED.

In the context of the present invention, LDL is defined as low density lipoprotein, the main transporter of cholesterol in plasma, and is composed of approximately 22% protein, 22% phospholipids, 8% cholesterol, 42% cholesterol esters, and 6% triglycerides. The main protein in LDL is apolipoprotein B-100 (apoB-100), a 550 kDa protein representing greater than 95% of the total protein mass of LDL, with smaller amounts of other LDL-associated apolipoproteins such as apoE, apoC-III, apoC-II, apoA-I, apoA-IV, apoM, apoJ, serum amyloid A4 (SAA4), calgranulin A, lysozyme C, apoD, apoH, α1-antitrypsin, orosomucoid-1, paraoxonase-1, retinol-binding protein, and prenylcysteine lyase-1.

In the context of the present invention oxidized LDL is defined as a wide range of LDL preparations which have been isolated from biological sources and which have in common the generation or presence of an oxidized particle. There are many *in vivo* mechanisms capable of oxidizing LDL including transition metals (iron and copper), and different enzyme systems such as lipoxygenases, ceruloplasmin, myeloperoxidase, NADPH oxidase and nitric oxide synthase. It can even be oxidized intracellularly in the lysosomes of macrophages.

### Methods of the invention

Another aspect of the invention relates to an *in vitro* method for obtaining data useful for predicting or prognosing the response to bariatric surgery in subjects with obesity, prior to the bariatric surgery, hereinafter referred to as the "first method of the invention", from a biological sample isolated from subcutaneous adipose tissue, comprising: a) quantifying the levels of expression of CYB5 and b) comparing the amounts obtained in step (a) with a reference amount of a group of subjects with obesity who are candidates to undergo bariatric surgery.

Preferably the first method of the invention further comprises quantifying in step a) the levels of expression of SYUG, HBA, GPX4, GRP78, SUCB2, LRP1, QCR1, SURF4 and AIFM1 and in step b) comparing the amounts obtained in step (a) of these biomarkers with a reference amount of a group of subjects with obesity who are candidates to undergo bariatric surgery.

In another preferred embodiment, steps (a) and/or (b) may be fully or partially automated.

Another aspect of the invention relates to an *in vitro* method for predicting or prognosing the response to bariatric surgery in subjects with obesity, prior to the bariatric surgery, hereinafter referred to as the "second method of the invention", which comprises the steps (a) - (b) according to the first method of the invention, and further comprising: c) assigning the individual who presents in an analysis the level of expression of CYB5 under-expressed with respect to the average reference values of a group of subjects with obesity who are candidates to undergo bariatric surgery, to the group of subjects presenting moderate response to bariatric surgery.

Preferably, the "second method of the invention" further comprises in step c) assigning the individual presenting in an analysis under-expressed levels of CYB5, SUCB2, QCR1, SURF4, GRP78, GPX4, AIFM1 and HBA, and overexpressed levels of LRP1 and SYUG, compared with respect to the average reference values of a group of subjects with obesity who are candidates to undergo bariatric surgery to the group of subjects presenting moderate response to bariatric surgery.

Another aspect of the invention relates to an *in vitro* method for obtaining useful data for predicting or prognosing the response to bariatric surgery in subjects with obesity, prior to the bariatric surgery, hereinafter referred to as the "third method of the invention", from an isolated biological sample of blood, serum, or plasma, comprising a) quantifying the levels of expression of oxidized LDL and b) comparing the amounts obtained in step (a) with a reference amount of a group of subjects with obesity who are candidates to undergo bariatric surgery.

Another aspect of the invention relates to an *in vitro* method for predicting or prognosing the response to bariatric surgery in subjects with obesity, prior to the bariatric surgery, hereinafter referred to as the "fourth method of the invention", which comprises steps (a) - (b) according to the third method of the invention, and further comprising c) assigning the individual presenting in an analysis the level of expression of oxidized LDL overexpressed with respect to the average reference values of a group of subjects with obesity who are candidates to undergo bariatric surgery to the group of subjects presenting moderate response to bariatric surgery.

The methods of the invention involve comparing the level of expression of biomarkers in isolated samples from subjects with the levels of expression of said biomarkers in a reference sample or with a median value.

In the context of the present invention, "reference sample" is understood as the sample which is used to determine the variation of the levels of expression of the biomarkers of the present invention. Depending on the selected biomarker, the reference sample will be taken from isolated samples from subcutaneous adipose tissue or from blood, serum or plasma.

"Reference value" is the level of expression of a biomarker of the invention in a reference sample. "Reference levels" may be determined by measuring levels of expression, and those reference levels may be adjusted to specific populations, e.g. a reference level may be related to age, so comparisons may be made between levels of expression in samples from subjects of a certain age and reference levels for a particular disease, phenotype, or lack thereof, in a certain age group. In a preferred embodiment, the reference value is obtained from the expression values obtained from a sample from individuals with obesity who are candidates to undergo bariatric surgery. Preferably, reference samples are taken from several individuals who are candidates to undergo bariatric surgery and are combined so that the reference value reflects the average value of these biomarkers in the population of individuals who are candidates to undergo bariatric surgery as previously defined herein. The expression profile in the reference sample can be generated from a population of 2, 3, 4, 5, 10, 15, 20, 30, 40, 50 or more individuals.

The detection of the levels of expression of CYB5, SYUG, HBA, GPX4, GRP78, SUCB2, LRP1, QCR1, SURF4, AIFM1 and oxidized LDL can be performed by any means known in the prior art. The levels of expression are to give a certain profile. The term "level of expression", also referred to as "product amount" or "amount of expression product", refers to the biochemical material.

The measurement of the quantity or concentration of expression product, preferably in a semi-quantitative or quantitative manner, can be carried out directly or indirectly. Direct measurement refers to the measurement of the amount or concentration of the expression product, which is directly correlated with the number of protein molecules. Such a signal (which may also be referred to as an intensity signal) may be obtained, for example, by measuring an intensity value of a chemical or physical property of such products. Indirect measurement includes the measurement obtained from a secondary component or a biological measurement system (e.g. the measurement of cellular responses, ligands, "tag" or enzymatic reaction products).

The term "amount," as used in the description, refers to, but is not limited to, the absolute or relative amount of the expression products, as well as any other value or parameter related thereto or derived from them. Said values or parameters comprise signal intensity values obtained from any of the physical or chemical properties of said expression products obtained by direct measurement. Additionally, said values or parameters include all those obtained by indirect measurement, e.g. any of the measurement systems described elsewhere in this document.

In another preferred embodiment of this aspect of the invention, the result, i.e. the level of expression of CYB5, SYUG, HBA, GPX4, GRP78, SUCB2, LRP1, QCR1, SURF4 and AIFM1, can be obtained by any method known in the prior art and specifically any of the following techniques: microarrays, Western blot, mass spectrometry or immunoassays such as ELISA, immunoblot, linear immunoassay (LIA), radioimmunoassay (RIA), immunofluorescence, x-map or protein chips. For immunoassays "marker compounds" may be used, which, as used in this description, refer to a compound capable of producing a chromogenic, fluorogenic, radioactive, and/or chemiluminescent signal that allows the detection and quantification of the amount of antibodies against the proteins of interest. The marker compound is selected from the list comprising radioisotopes, enzymes, fluorophores, or any molecule capable of being conjugated with another molecule or detected and/or quantified directly. This marker compound may be bound to the antibody directly or through another compound. Some examples of marker compounds that bind directly are, but are not limited to, enzymes such as alkaline phosphatase or peroxidase, radioactive isotopes such as 32P or 35S, fluorochromes such as fluorescein or metal particles, for direct detection by colorimetry, autoradiography, fluorimetry, or metallography, respectively.

In the method of the present invention, the expression of the proteins may be normalized, preferably, in relation to the expression of another protein. Methods of normalization are well known in the prior art.

In order to detect and quantify oxidized LDL several techniques exist in the prior art among which are highlighted ELISA technique using two monoclonal antibodies for different epitopes against Apo B, quantification by means of a competitive ELISA, detection by means of immunofluorescence in cell monolayers, spectrophotometric measurement at 232 nm where the absorbance at 232 nm is compared to the absorbance of native LDL.

The term "comparison" as used in the description, refers to, but is not limited to, the comparison of the amount of the expression products of CYB5, SYUG, HBA, GPX4, GRP78, SUCB2, LRP1, QCR1, SURF4, AIFM1 and oxidized LDL from the biological sample to be analyzed, also called problem biological sample, with an amount of the expression products of CYB5, SYUG, HBA, GPX4, GRP78, SUCB2, LRP1, QCR1, SURF4, AIFM1 and oxidized LDL from one or more desirable reference samples. The reference sample may be analyzed, for example, simultaneously or consecutively, along with the problem biological sample.

Once the levels of expression relative to the reference values have been determined, it is necessary to identify whether there are any alterations in expression (increase or decrease in expression). Expression (and levels of the gene expression product) is considered increased in a sample of the subject matter of study when the levels of increase relative to the reference sample are at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more. Similarly, expression is considered decreased when its levels decrease relative to the reference sample by at least 5%, at least 10%, at least 15%, at least 20%, at least 25%, at least 30%, at least 35%, at least 40%, at least 45%, at least 50%, at least 55%, at least 60%), at least 65%, at least 70%, at least 75%, at least 80%, at least 85%, at least 90%, at least 95%, at least 100% (i.e. absent).

The calculation of said comparison in the methods of the present invention may be performed manually or computer-assisted.

The method of the present invention can be applied to samples from individuals of any sex, i.e., male or female, and of any age.

The invention provides a method for assigning a human subject to one of two groups: group 1, comprising subjects identifiable by the method of the invention, and group 2, representing the remaining subjects.

It is possible to provide a personalized therapy to an individual depending on whether the individual is assigned to group 1 or group 2. Group 1 comprises subjects identifiable by the method of the invention as individuals presenting moderate response to bariatric surgery and group 2 represents the remaining subjects.

### Kit or composition of the invention and uses

Another aspect of the invention relates to a kit or device, hereinafter referred to as the kit or device of the invention, which comprises the elements necessary to quantify the level of expression of CYB5. Preferably, it comprises the elements necessary to simultaneously quantify CYB5, SYUG, HBA, GPX4, GRP78, SUCB2, LRP1, QCR1, SURF4 and AIFM1.

Another aspect of the invention relates to a kit or device comprising the elements necessary to quantify oxidized LDL. In a preferred embodiment the kit or device of the invention comprises: (a) means for detecting in a biological sample obtained from the subject the levels of expression of the biomarkers of the invention, (b) means for comparing the level of expression the biomarkers determined in (a) with a reference sample, and (c) instructions for a medical professional to prescribe bariatric surgery only to those subjects who can be classified in the good response group.

The kit or device of the invention may be used and the use is not particularly limited, although use in the method of the invention in any of its embodiments is preferred. The kit or device of the invention may comprise controls, program instructions and information necessary to carry out any of the methods of the invention.

Automation of the method of the invention by implementing it in a computer program
Another aspect of the invention relates to a computer program comprising instructions for performing the method according to any of the methods of the invention.

In particular, the invention encompasses computer programs arranged on or within a carrier. The carrier may be any entity or device capable of supporting the program. When the program is incorporated into a signal that may be transported directly by a cable or other device or means, the carrier may be constituted by said cable or other device or means. As a variant, the carrier could be an integrated circuit in which the program is included and which has been adapted to execute, or to be used in the execution of the corresponding processes.

For example, the programs could be incorporated into a storage medium, such as a ROM memory, a CD ROM memory or a semiconductor ROM memory, a USB memory, or a magnetic recording medium, for example, a floppy disk or a hard disk. Alternatively, the programs could be supported on a transmissible carrier signal; for example, it could be an electrical or optical signal that could be transported via electrical or optical cable, by radio, or by any other means.

The invention also extends to computer programs adapted so that any processing means can carry out the methods of the invention. Such programs may be in the form of source code, object code, an intermediate source of object code and code, for example, as in partially compiled form, or in any other form suitable for use in implementing the processes according to the invention. Computer programs also encompass cloud applications based on such a procedure.

Other aspects of the invention relate to the readable storage medium and the transmissible signal comprising program instructions necessary for the execution of the method of the invention by a computer.

Thus, another aspect of the invention relates to a computer readable storage medium comprising program instructions capable of causing a computer to carry out the steps of any of the methods of the invention.

Throughout the description and claims the word "comprise" and its variants are not intended to exclude other technical features, additives, components or steps. For those skilled in the art, other objects, advantages and features of the invention will be apparent in part from the description and in part from the practice of the invention. The following examples and drawings are provided for illustration purposes only and are not intended to be limiting of the present invention.

### BRIEF DESCRIPTION OF THE FIGURES

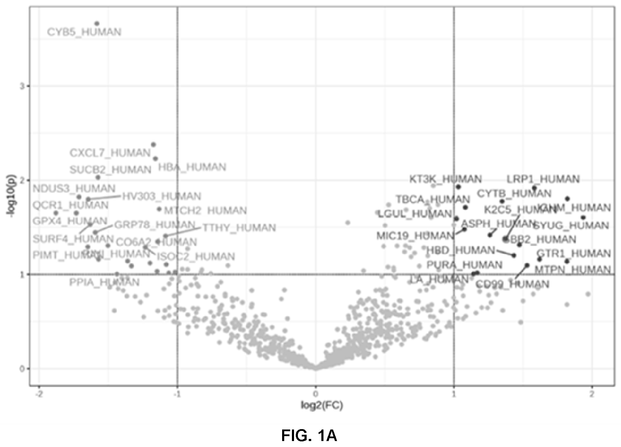

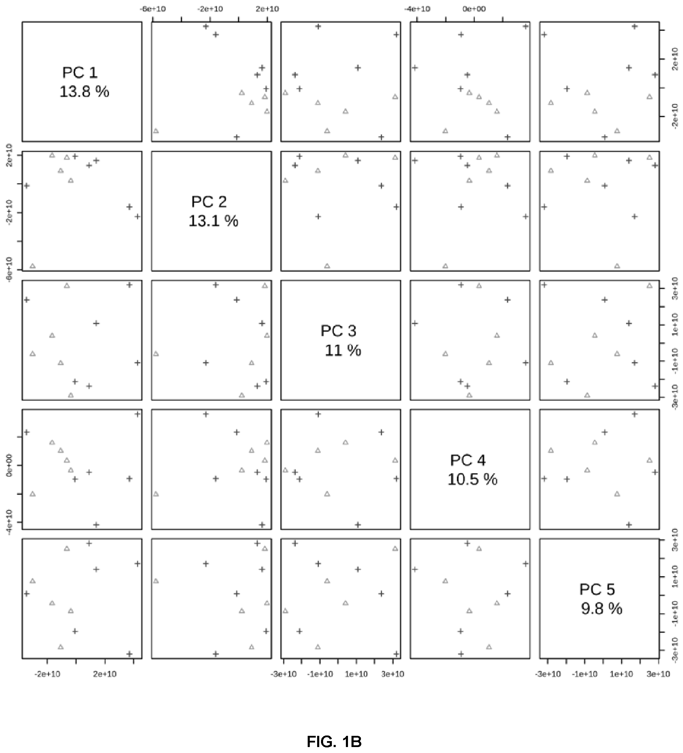

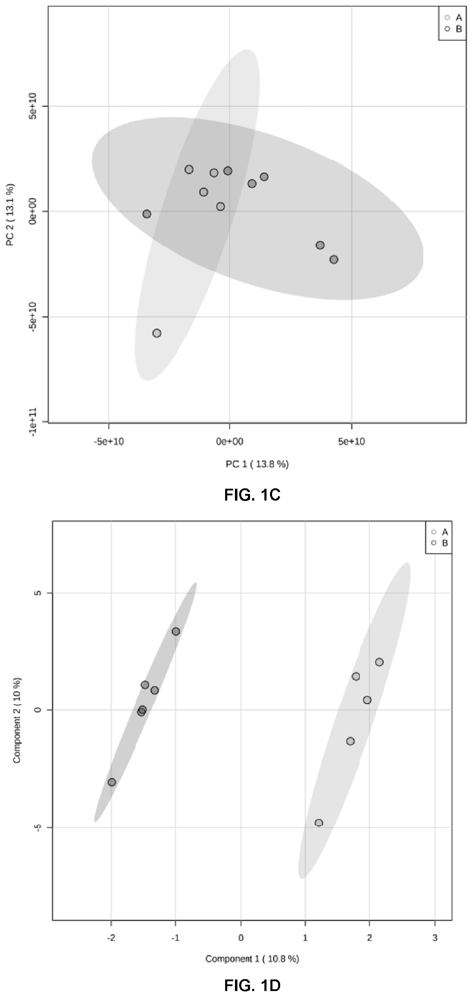

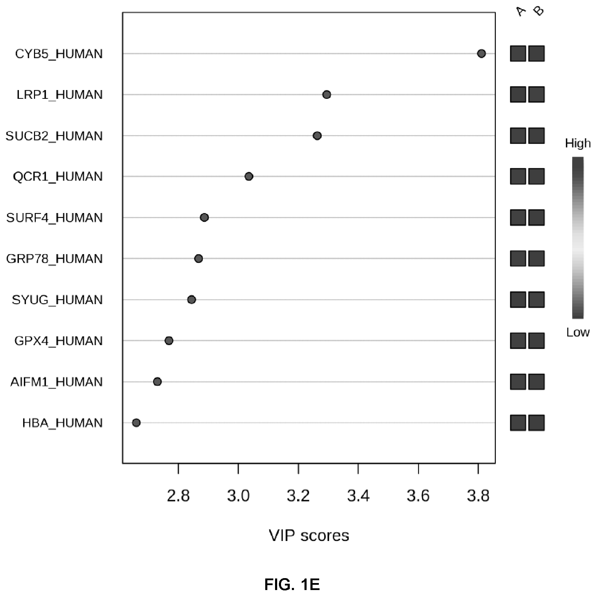

**Figure 1****:** Multivariate analysis of the proteomic data from study subjects. (A) Featured proteins obtained by plot volcano. Proteins overexpressed in the moderate response vs. good response group are shown in red, and proteins under-expressed are shown in blue. (B) Pairwise score plots between the main PC (principal components) which were obtained by PCA (principal component analysis). The variances of each PC are found in each diagonal box. (C) Score plots obtained by PCA between the first 2 PC. Variances are shown in parentheses. (D) Score plot obtained by PLS-DA (partial least squares discriminant analysis). Variances are shown in parentheses (E) VIP score of proteins with differential expression in subcutaneous adipose tissue as possible biomarker candidates. 10 proteins. A = Good response; B = Moderate response to bariatric surgery. Red: overexpression, Blue: under-expression.
**Figure 2****:** Analysis of possible markers of predictive response of bariatric surgery. (A) ROC curve for individual proteins obtained by VIP score by the classic univariate method. (B) ROC curve for proteins obtained by VIP score jointly using the multivariate method. (C) Average probabilities and (D) accuracy value (0.976) performed in 100 cross validations.
**Figure 3****:** Scatter plot showing levels of expression of CYB5 in the group of subjects with a good response to bariatric surgery (shown in red) and in the group with moderate response (shown in green).
**Figure 4****:** Correlation studies: negative correlation between plasma levels of expression of oxidized LDL and CYB5 protein prior to bariatric surgery.

### DETAILED DESCRIPTION OF THE INVENTION

**Analysis of the proteome of subcutaneous adipose tissue from morbidly subjects with obesity undergoing bariatric surgery to identify predictive markers of weight loss response.**

### Subjects

This study it was carried out with a cohort of subjects with obesity who underwent bariatric surgery. The subjects (n=12) were monitored before (PRE) and after (POST) the intervention.

Subjects were recruited at the Surgery Unit of the Virgen del Rocío University Hospital (Seville). All of them signed the informed consent, which was approved by the corresponding Ethics Committee.

Inclusion criteria for the study were ages between 18 and 65 years and BMI >40 kg/m², or BMI >35 kg/m² and comorbidities compromised by the presence of obesity itself, such as diabetes, hypertension, dilated cardiomyopathy or sleep apnea.

Exclusion criteria were the existence of severe psychiatric disorder, pregnancy, chronic or acute inflammatory disease, oncological disease, asthma or alcohol and/or drug abuse history.

Anthropometric and biochemical parameters of the subjects were analyzed before and 16 weeks after the intervention, and subcutaneous adipose tissue biopsies were performed at these times to carry out a quantitative proteomic study (LC-MS/MS + SWATH) in order to analyze the state of this tissue and identify possible proteins that would allow the response to bariatric surgery to be discriminated.

The subjects' response was also analyzed 15 months after the intervention in order to establish the assessment criteria for dividing the subjects into those with a good response and those with moderate response. As criterion for improvement of response, the loss of body mass index was determined after intervention, as this is the most standardized parameter as an indicator of response to bariatric surgery. A good response to bariatric surgery was considered to be a decrease in body mass index (BMI) greater than the median BMI loss of subjects undergoing bariatric surgery 15 months after the intervention. In the case of this group of subjects, the median BMI loss compared to the baseline was 31.31%. Thus, two groups of subjects were established: good response with a BMI loss >30% and moderate response with a BMI loss <30%.

### Characterization of study subjects before and after bariatric surgery

Subjects showed an improvement in the main anthropometric and metabolic parameters after the intervention (Table 2).

**Table 2: Anthropometric and biochemical parameters of the study subjects before and after bariatric surgery.**

| | **PRE-BS** | **POST-BS** | **p value** |
|---|---|---|---|
| Number of patients | 12 | | |
| Age (years) | 43.7 ±3.5 | | |
| Sex (f/m) | 2/10 | | |
| TIO (years) | 24±2.7 | | |
| BMI (kg/m²) | 51.6±5.8 | 34±5.7 | <0.0001 |
| Waist perimeter (cm) | 134.1±17.4 | 99.2±13.7 | <0.0001 |
| Hip perimeter (cm) | 150.1±15.4 | 118.7±14.3 | <0.0001 |
| WHR | 0.9±0.1 | 0.8±0.8 | 0.017 |
| Glocose (mg/dL) | 103.6±36.8 | 79.7±8.9 | 0.025 |
| Insuline (mU/L) | 23.5±8.7 | 8±4.9 | <0.0001 |
| HOMA | 6.5±5 | 1.6±1 | 0.004 |
| HbA1c | 6.2±1.1 | 5.3±0.3 | 0.007 |
| Total cholesterol (mg/dL) | 196.431.9 | 188.3±25.1 | 0.354 |
| Triglycerides (mg/dL) | 120.4±28 | 79±21.8 | 0.002 |
| HDL (mg/dL) | 45.6±7.3 | 58.4±11.8 | 0.002 |
| LDL (mg/dL) | 128.5±32.8 | 113.6±23.6 | 0.070 |
| sTNFR2s (mg/ml) | 5.5±1.4 | 5±0.9 | 0.225 |
| CRP (Us ng/L) | 16.4±14.7 | 2.6±2.6 | 0.005 |
| LDL oxidase (ng/ml) | 187.4±165 | 112.8±66.3 | 0.170 |
| Adiponectine (ng/ml) | 4769.9±2128.5 | 9075.9±3583.7 | 0.001 |
| Body fat | 71±13 | 37±11.2 | 0.006 |
| Body muscle | 57.6±4.9 | 49.5±3.5 | 0.012 |
| Total water | 45.2±4.6 | 37.5±3 | 0.011 |
| Bone mass | 3.1±0.2 | 2.6±0.2 | 0.010 |
| Basal metabolic rate | 8282.8±908.7 | 6739.6±518.3 | 0.009 |

Values represent the mean ± standard deviation for each parameter. P-value obtained by t-test for paired samples. BS, bariatric surgery; BMI, body mass index; WHR, waist-to-hip ratio; HOMA, homeostatic model assessment; HbA1c, glycosylated hemoglobin; HDL, high density lipoprotein; LDL, low density lipoprotein; sTNFR2s, serum levels of soluble TNF-α 2 receptor; CRP, C-reactive protein.

BMI and total fat mass decreased significantly (-34.1% and -47.9%, respectively). Parameters related to carbohydrate metabolism (glucose, insulin, HOMA, HbA1c) also showed significant improvement, with an average drop of 23.1%, 66%, 75% and 14.5% respectively. In addition, 66% of subjects with diabetes experienced a remission of such pathology. Improvements in the lipid profile were less pronounced. Thus, HDL increased by 28% and triglycerides decreased by 34.4%. However, neither total cholesterol nor LDL, which is an important cardiovascular risk factor, showed significant improvement in their levels.

### SWATH proteomic analysis of subcutaneous adipose tissue

A quantitative proteomic study of the subcutaneous adipose tissue of 12 of the individuals of the cohort of initial subjects with obesity was performed both before (PRE) and after (POST) bariatric surgery, using the SWATH methodology (sequential window acquisition of all theoretical fragment ion spectra mass spectrometry).

### Biopsy isolation and protein extraction

Adipose tissue biopsies were obtained from subjects before and after bariatric surgery that were washed in saline solution, and immediately frozen at -80°C until use.

### Identification and quantification of proteins expressed in the biopsied tissue

Biopsies were subsequently processed for protein extraction. For this purpose, the samples (50 mg of tissue) were homogenized in 200 microliters of lysis buffer with a Teflon homogenizer and subsequent sonication. Finally, the samples were centrifuged and the fat-free aqueous fraction was obtained, which was quantified and adjusted for subsequent digestion with trypsin to be identified by mass spectrometry by liquid chromatography coupled with tandem mass spectrometry (LC-MS/MS) and the SWATH technique (sequential window acquisition of all theoretical fragment ion spectra mass spectrometry). This SWATH technique uses an MS/MS independent data acquisition method to generate systematically complete, high specificity ion fragment maps, which can be consulted to determine the presence and quantify any protein of interest, using a target data analysis strategy.

### Bioinformatics analysis

To analyze the proteins and to be able to classify them according to their ontological categories (Gene Ontology), the Panther Classification System web platform (http://pantherdb.org/) was used. To carry out the predictive marker study the MetaboAnalyst platform (https://www.metaboanalyst.ca) was used, a free web platform dedicated to the statistical, functional, and integrative analysis of omic data for the search for biomarkers.

### Correlation studies

Linear correlation studies were performed between the expression of proteins of interest and the anthropometric and biochemical parameters available using IBM SPSS Statistics 25 statistical analysis software.

### Identification of biomarkers

A proteomic analysis was performed in order to identify potential preoperative response markers. For this, statistical analysis was performed using the MetaboAnalyst platform in order to identify possible biomarkers. A first exploratory analysis was carried out where a total of 930 proteins were identified in the adipose tissue proteome, of which 156 modified their expression significantly after bariatric surgery.

For the identification of predictive biomarkers of response to bariatric surgery the MetaboAnalyst web platform was used. A decrease in body mass index (BMI) above the median of the cohort of subjects (31.31%) 15 months after surgery was considered as a good response to bariatric surgery. Based on these criteria, our study subjects were distributed in two groups: those with a good response and those with a poor response to bariatric surgery.

The volcano plot (Figure 1A) shows the most relevant proteins (fold change threshold/t-tests threshold >0.1). The discriminant analysis (Figure 1D) (Partial Least Squares - Discriminant Analysis (PLS-DA) showed a clear separation of the groups and a top 10 of the best components determined by the Variable Importance in Projection (VIP) value (Figure 1E), which estimates the importance of each variable in the projection used in a PLS model (partial least squares regression).

First, a univariate analysis of the differential expression of proteins between the two study groups was performed. This type of methods allows obtaining a preliminary view on possible proteins that may be of interest. A fold change (FC) threshold value of 2 was set to perform the selection of the proteins of interest. In order to make an accurate selection of the potential biomarkers, the volcano plot technique was used (Figure 1A), which allows relating the FC of the proteins together with their significance value, obtained by Student's t-tests. A value p 0.1 was selected as the threshold.

42 proteins exceeded said threshold. Among them CYB5 (log2 (FC) = 1, 5824; log10 (p) = 3.6626) stood out in particular, as it was the protein that exhibited the best combination of magnitude values and significance of change of expression between groups.

Next, a principal component analysis (PCA) was performed. This technique helps to group a large number of correlated variables into a significantly smaller number of them, determining the so-called scores. These scores are reordered with the aim of reducing variability, forming the so-called principal components (PC). The first PC would contain as much of the possible variability of the sample, with the rest being distributed among the successive PCs. Figure 1B shows the most important PCs of our study together with their variance, which represents the contribution of each of them to the separation between groups. PCA allows to identify differences between groups and which subjects contributed to a greater extent to that difference, which is extremely important for the development of predictive models. Figure 1C shows the existence of two groups: group A, in green, representing the group of subjects who had a good response to bariatric surgery, and group B, in red, which is the group of subjects with a suboptimal response to the surgery. These graphs reveal the existence of two partially differentiated groups. To enhance this differentiation, techniques such as partial least squares discriminant analysis (PLS-DA) were used. This is a supervised technique consisting of the combination of PCs obtained through PCA with multivariable regression models specifically designed to maximize the covariance between the groups established in the study design, as shown in Figure 1D. The main contribution of the PLS-DA technique to this biomarker search study was the creation of a list of prominent proteins based on a VIP score. Its objective was to determine, by calculating the projection of a certain variable within its respective component, those proteins whose presence may allow to better predict the group to which a certain subject will belong.

As shown in Figure 1E, the protein with the highest VIP score was CYB5, coinciding with the result obtained in the univariate analysis.

These results confirmed CYB5 as the main protein to be considered as a possible biomarker.

To deepen more in the capacity of this protein as a possible marker, the next step was the elaboration and analysis of ROC (receiver operating characteristic curve), univariate curves that allow evaluating the prognostic accuracy of a biomarker. They consist of a graphical representation of the relationship between sensitivity with complementary specificity. They allow calculating an index called the area under the curve (AUC), which constitutes a good measure of the overall validity of a biomarker.

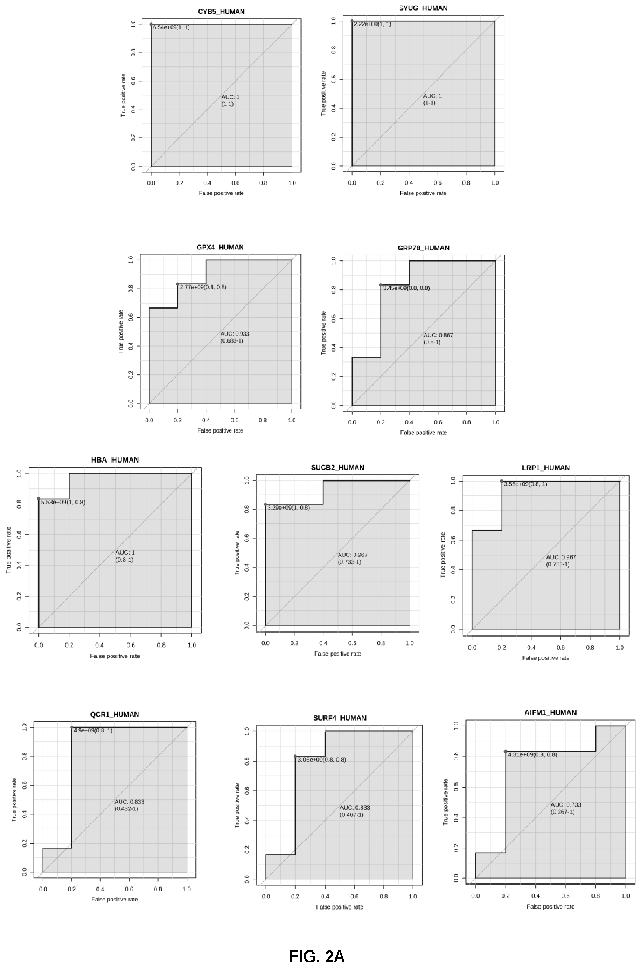

The top 10 VIP score candidates were subjected to this analysis. As shown in Figure 2A, the proteins with the best predictive capacity would be SYUG and CYB5 (AUC=1), while the rest of the candidates was placed in an AUC>0.7, indicating a good discriminatory capacity for all of them.

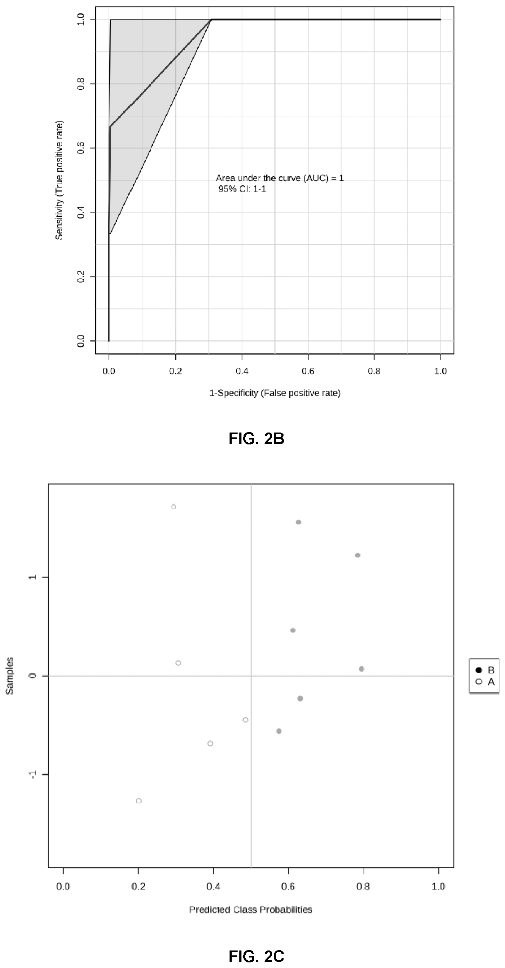

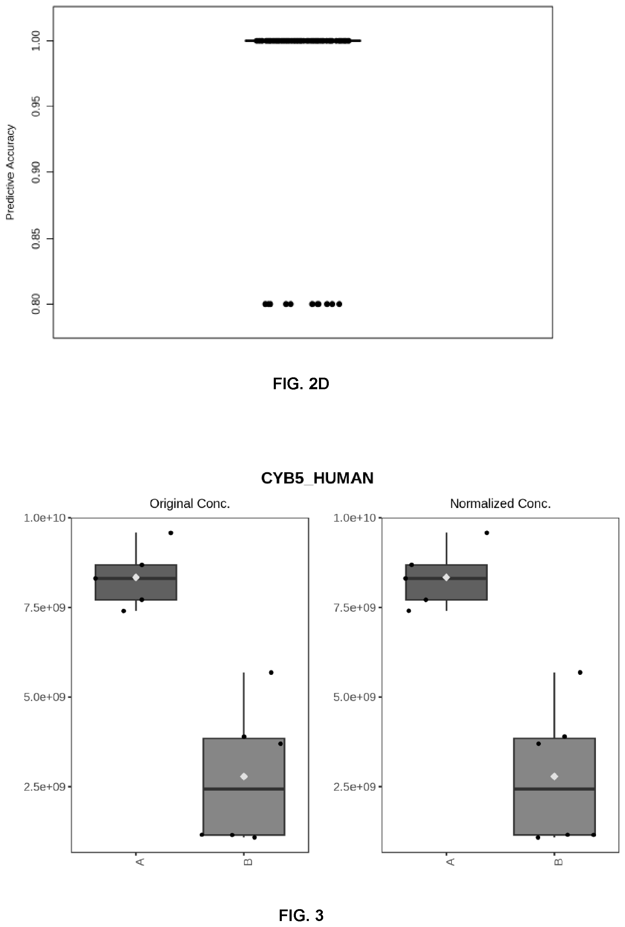

This analysis was completed with a multivariate ROC curve analysis including the 10 possible candidates (Figure 2B). In this case, a ROC curve of 1 was observed, demonstrating a good discriminatory capacity of the different groups, as observed on the average of the predicted class probabilities of each sample in the 100 cross-validations (Figure 2C). In addition, an accuracy value of 0.976 was determined (Figure 2D).

The application of this panel of 10 proteins provides a high discriminatory power, allowing accurate and effective prediction of responsiveness of subjects prior to bariatric surgery.

These results show that CYB5 is a clear biomarker candidate. The CYB5 protein, or cytochrome b5, is an oxidoreductase type protein located in the outer mitochondrial membrane whose function is to transfer electrons within the electron transport chain. Its expression in both groups is shown in Figure 3.

On the other hand, the panel of molecular biomarkers of response to predictive bariatric surgery, consisting of the 10 top VIP score biomarkers, evidences the role of the initial state of adipose tissue as key to a good improvement.

It is therefore concluded that the previous state of the subjects' subcutaneous adipose tissue could be critical in the response derived from bariatric surgery, being a possible parameter to be evaluated in clinical practice.

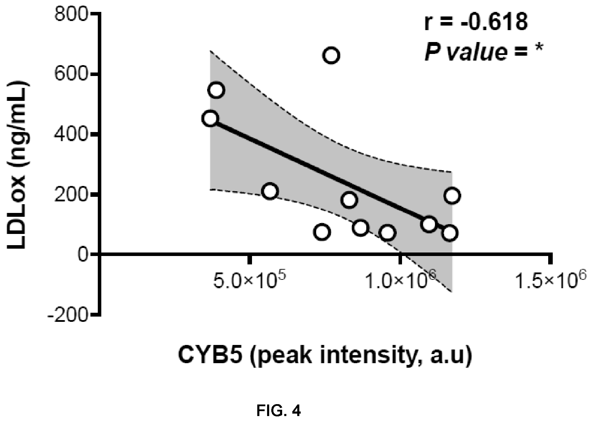

However, because these proteins should be measured in adipose tissue, obtaining the sample would require an invasive method. Therefore, a correlation study with anthropometric and/or plasma parameters was performed in order to be able to reduce invasiveness and identify a surrogate biomarker for the expression of these proteins that is more easily measurable. In this regard, it was observed that plasma oxidized LDL values were significantly inversely correlated with CYB5 peak intensity levels in the subcutaneous adipose tissue proteome (r=-0.618; p=0.043) (Figure 4).

Thus, oxidized LDL, a lipoprotein related to the onset of oxidative stress, could be indicative of the state of adipose tissue prior to surgery and therefore be a possible predictive marker of response to bariatric surgery.

The CYB5 protein highlights as a predictive biomarker of response to bariatric surgery, which further correlates with plasma levels of oxidized LDL, which can be used as a surrogate marker of response to bariatric surgery in blood samples.

## Claims

1. *In vitro* use of the level of expression of CYB5 in a sample of subcutaneous adipose tissue from a subject with obesity candidate to undergo bariatric surgery, isolated prior to bariatric surgery, as a biomarker for predicting or prognosing the response to bariatric surgery, where under-expressed levels with respect to the average reference values of a group of with obesity who are candidates to undergo bariatric surgery is an indicator of belonging to the group of subjects presenting moderate response to bariatric surgery.

2. The *in vitro* use of the level of expression of CYB5 according to the previous claim, **characterized in that** it is used in combination with the level of expression of SYUG, HBA, GPX4, GRP78, SUCB2, LRP1, QCR1, SURF4, and AIFM1, all measured in a sample of subcutaneous adipose tissue from a subject with obesity, isolated prior to bariatric surgery, where under-expressed levels of CYB5, SUCB2, QCR1, SURF4, GRP78, GPX4, AIFM1, and HBA, and overexpressed levels of LRP1 and SYUG compared to the average reference values of a group of subjects with obesity who are candidates to undergo bariatric surgery are indicators of belonging to the group of subjects presenting moderate response to bariatric surgery.

3. *In vitro* use of the level of expression of oxidized LDL in an isolated blood, serum, or plasma sample from a subject with obesity as a surrogate biomarker for CYB5 for predicting or prognosing the response to bariatric surgery, where overexpressed levels compared to the average reference values of a group of subjects with obesity who are candidates to undergo bariatric surgery are an indicator of belonging to the group of subjects presenting moderate response to bariatric surgery.

4. *In vitro* method for obtaining useful data for predicting or prognosing the response to bariatric surgery in subjects with obesity, prior to bariatric surgery, from a biological sample isolated from subcutaneous adipose tissue, comprising:
a) quantifying level of expression of CYB5.
b) comparing the amounts obtained in step (a) with a reference amount of a group of subjects with obesity who are candidates to undergo bariatric surgery.

5. The *in vitro* method for obtaining useful data according to the previous claim, which also comprises quantifying in step a) the levels of expression of SYUG, HBA, GPX4, GRP78, SUCB2, LRP1, QCR1, SURF4, and AIFM1 and in step b) comparing the quantities obtained in step (a) of these biomarkers with a reference quantity of a group of subjects with obesity who are candidates for bariatric surgery.

6. *In vitro* method for predicting or prognosing the response to bariatric surgery in subjects with obesity, prior to bariatric surgery, comprising steps (a) - (b) of claim 4, and further comprising:
c) assigning the individual who presents in an analysis the level of expression of CYB5 that is under-expressed with respect to the average reference values of a group of subjects with obesity who are candidates for bariatric surgery, to the group of subjects presenting moderate response to bariatric surgery.

7. *In vitro* method for predicting or prognosing the response to bariatric surgery in subjects with obesity, prior to bariatric surgery, comprising steps (a) - (b) of claim 5, and further comprising:
c) assigning the individual who presents in an analysis under-expressed levels of CYB5, SUCB2, QCR1, SURF4, GRP78, GPX4, AIFM1, and HBA, and overexpressed levels of LRP1 and SYUG with respect to the average reference values of a group of subjects with obesity who are candidates for bariatric surgery, to the group of subjects presenting moderate response to bariatric surgery.

8. *In vitro* method for obtaining useful data for predicting or prognosing the response to bariatric surgery in subjects with obesity, prior to bariatric surgery, from an isolated biological sample of blood, serum, or plasma, comprising:
a) quantifying the levels of expression of oxidized LDL.
b) comparing the amounts obtained in step (a) with a reference amount of a group of subjects with obesity who are candidates to undergo bariatric surgery.

9. *In vitro* method for predicting or prognosing the response to bariatric surgery in subjects with obesity prior to bariatric surgery, comprising steps (a) - (b) of claim 8, and further comprising:
c) Assigning individuals who, in an analysis, show overexpressed levels of oxidized LDL compared to the average reference values of a group of subjects with obesity who are candidates for bariatric surgery, to the group of subjects presenting moderate response to bariatric surgery.
